(19) Europäisches Patentamt
European Patent Office
Office européen des brevets

(11) Veröffentlichungsnummer: **0 438 686 A2**

(12) **EUROPÄISCHE PATENTANMELDUNG**

(21) Anmeldenummer: **90123613.3**

(22) Anmeldetag: **08.12.90**

(51) Int. Cl.5: **C07D 249/08**, A01N 43/653, C07D 303/22, C07C 49/84, C07C 49/577, C07C 259/08, C07C 62/08

(30) Priorität: **21.12.89 DE 3942417**

(43) Veröffentlichungstag der Anmeldung: **31.07.91 Patentblatt 91/31**

(84) Benannte Vertragsstaaten: **BE CH DE FR GB IT LI NL**

(71) Anmelder: **BAYER AG**
**W-5090 Leverkusen 1 Bayerwerk(DE)**

(72) Erfinder: **Scherkenbeck, Jürgen, Dr.**
**Auf dem Bruch 49**
**W-5090 Leverkusen 3(DE)**
Erfinder: **Stroech, Klaus, Dr.**
**Rolsberger Strasse 22**
**W-5650 Solingen 19(DE)**
Erfinder: **Fugmann, Burghard, Dr.**
**Ellenbeek 31**
**W-5603 Wülfrath(DE)**
Erfinder: **Dutzmann, Stefan, Dr.**
**Leinenweberweg 33**
**W-4000 Düsseldorf 13(DE)**

(54) **Triazolylmethyl-cyclopropyl-Derivate.**

(57) Triazolylmethyl-cyclopropyl-Derivate der Formel

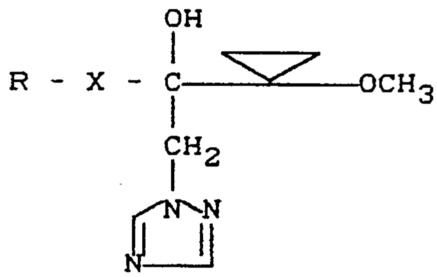

(I),

in welcher
R und X die in der Beschreibung angegebenen Bedeutungen haben,
sowie deren Säureadditions-Salze und Metallsalz-Komplexe,
ein Verfahren zur Herstellung der neuen Stoffe und deren Verwendung als Fungizide.

Neue Zwischenprodukte, Verfahren zu deren Herstellung und deren Verwendung zur Synthese von Triazolylmethyl-cyclopropyl-Derivaten der Formel (I).

EP 0 438 686 A2

## TRIAZOLYLMETHYL-CYCLOPROPYL-DERIVATE

Die vorliegende Anmeldung betrifft neue Triazolylmethyl-cyclopropyl-Derivate, ein Verfahren zu deren Herstellung und deren Verwendung als Fungizide.

Es ist bereits bekannt geworden, daß bestimmte Azolylmethyl-cyclopropyl-Derivate fungizide Eigenschaften besitzen (vgl. EP-OS 0 180 136 und EP-OS 0 297 345). So können z.B. 1-(4-Phenyl-phenyl)-1-(1-methylthio-cyclopropyl)-2-(1,2,4-triazol-1-yl)-ethan-1-ol, 1-(4-Chlor-phenyl)-1-(1-methylthio-cyclopropyl)-2-(1,2,4-triazol-1-yl)-ethan-1-ol, 1-(4-Fluor-phenyl)-1-(1-methylthio-cyclopropyl)-2-(1,2,4-triazol-1-yl)-ethan-1-ol und 1-(2,4-Difluor-phenyl)-1-(1-methylthio-cyclopropyl)-2-(1,2,4-triazol-1-yl)-ethan-1-ol zur Bekämpfung von Pilzen eingesetzt werden. Die Wirksamkeit dieser Stoffe ist gut; sie läßt allerdings bei niedrigen Aufwandmengen in manchen Fällen zu wünschen übrig.

Es wurden nun neue Triazolylmethyl-cyclopropyl-Derivate der Formel

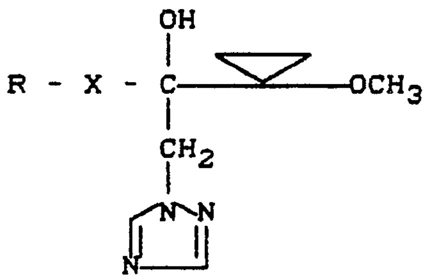

(I),

in welcher

R für die Reste der Formeln

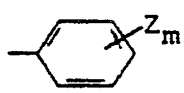

oder

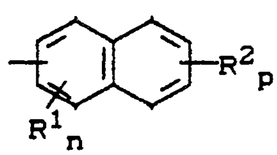

steht, worin

Z für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht und

m für die Zahlen 0, 1, 2 oder 3 steht,

$R^1$ für Halogen, Alkyl mit 1 bis 4 Kohlenstoffatomen oder Alkoxy mit 1 bis 4 Kohlenstoffatomen steht,

n für die Zahlen 0, 1 oder 2 steht,

$R^2$ für Halogen, Alkyl mit 1 bis 4 Kohlenstoffatomen oder Alkoxy mit 1 bis 4 Kohlenstoffatomen steht und

p für die Zahlen 0, 1 oder 2 steht, und

X für $CH_2$ oder eine direkte Bindung steht, sowie deren Säureadditions-Salze und Metallsalz-Komplexe gefunden.

Weiterhin wurde gefunden, daß man Triazolylmethyl-cyclopropyl-Derivate der Formel (I) sowie deren Säureadditions-Salze und Metallsalz-Komplexe erhält, wenn man Oxirane der Formel

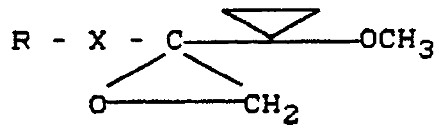

(II),

in welcher
R und X die oben angegebenen Bedeutungen haben,
mit 1,2,4-Triazol der Formel

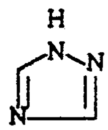

(III),

gegebenenfalls in Gegenwart eines Säurebindemittels und in Gegenwart eines Verdünnungsmittels umsetzt und gegebenenfalls anschließend an die so erhaltenen Verbindungen der Formel (I) eine Säure oder ein Metallsalz addiert.

Schließlich wurde gefunden, daß die neuen Triazolylmethyl-cyclopropyl-Derivate der Formel (I) sowie deren Säureadditions-Salze und Metallsalz-Komplexe sehr gute fungizide Eigenschaften besitzen.

Die erfindungsgemäßen Stoffe enthalten ein asymmetrisch substituiertes Kohlenstoffatom. Sie können daher in optischenIsomerenformen anfallen. Die vorliegende Erfindung betrifft sowohl die einzelnen Isomeren als auch deren Gemische.

Überraschenderweise besitzen die erfindungsgemäßen Stoffe eine bessere fungizide Wirksamkeit als das 1-(4-Phenyl-phenyl)-1-(1-methylthio-cyclopropyl)-2-(1,2,4-triazol-1-yl)-ethan-1-ol, das 1-(4-Chlor-phenyl)-1-(1-methylthio-cyclopropyl)-2-(1,2,4-triazol-1-yl)-ethan-1-ol, das 1-(4-Fluor-phenyl)-1-(1-methylthio-cyclopropyl)-2-(1,2,4-triazol-1-yl)-ethan-1-ol und das 1-(2,4-Difluor-phenyl)-1-(1-methylthio-cyclopropyl)-2-(1,2,4-tria zol-l-yl)-ethan-1-ol, welches konstitutionell ähnliche, vorbekannte Wirkstoffe gleicher Wirkungsrichtung sind.

Die erfindungsgemäßen Triazolylmethyl-cyclopropyl-Derivate sind durch die Formel (I) allgemein definiert. In dieser Formel stehen vorzugsweise

R für die Reste der Formeln

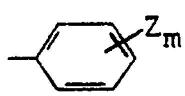

,

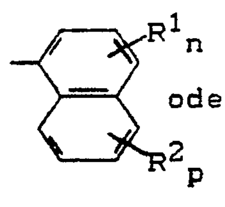

oder

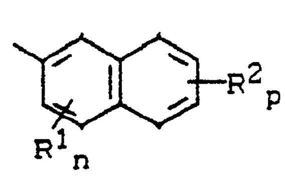

,

worin

Z für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/ oder Methyl substituiertes Phenyl oder für gegebenenfalls durch Fluor, Chlor und/oder Methyl substituiertes Phenoxy steht,

m für die Zahlen 0, 1, 2 oder 3 steht,

$R^1$ für Fluor, Chlor, Methyl, Ethyl, Methoxy oder Ethoxy steht,

m für die Zahlen 0, 1 oder 2 steht,

$R^2$ für Fluor, Chlor, Methyl, Ethyl, Methoxy oder Ethoxy steht und

p für die Zahlen 0, 1 oder 2 steht, und

X für $CH_2$ oder eine direkte Bindung.

Wenn m für die Zahlen 2 oder 3 steht, können die für Z stehenden Reste gleich oder verschieden sein.

Ebenso können die für $R^1$ bzw. $R^2$ stehenden Reste gleich oder verschieden sein, wenn n bzw. p für 2 stehen.

Bevorzugte erfindungsgemäße Stoffe sind auch Additionsprodukte aus Säuren und denjenigen Triazolylmethyl-cyclopropyl-Derivatender Formel (I), in denen R und X die Bedeutungen haben, die bereits vorzugsweise für diese Reste genannt wurden.

Zu den Säuren, die addiert werden können, gehören vorzugsweise Halogenwasserstoffsäuren, wie z.B.

die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen Triazolylmethyl-cyclopropyl-Derivaten der Formel (I), in denen R und X die Bedeutungen haben, die bereits vorzugsweise für diese Reste genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für Triazolylmethyl-cyclopropyl-Derivate der Formel (I) seien die in der folgenden Tabelle aufgeführten Stoffe genannt.

<u>Tabelle</u>

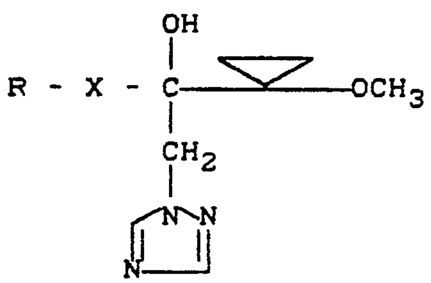

(I),

| R | X |
|---|---|
| 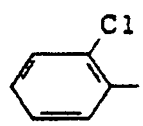 | $CH_2$ |
| 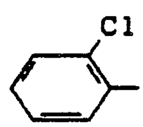 | - |
| 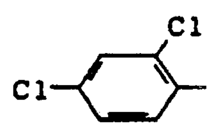 | $CH_2$ |
| 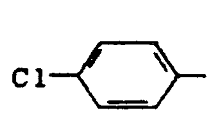 | $CH_2$ |

Tabelle (Fortsetzung)

| R | X |
|---|---|
| 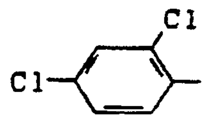 | - |
| 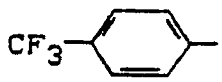 | $CH_2$ |
| 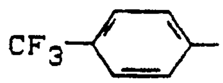 | - |
| 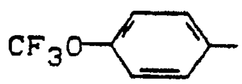 | $CH_2$ |
| 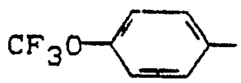 | - |
| 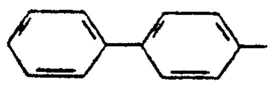 | $CH_2$ |
| 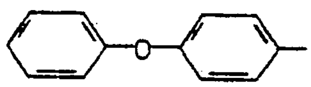 | - |
| 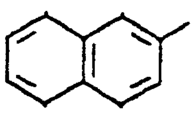 | - |
| 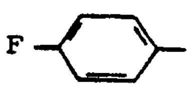 | - |
| 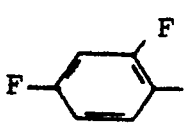 | $CH_2$ |

Verwendet man 2-(4-Fluor-benzyl)-2-(1-methoxy-cyclopropyl)-oxiran und 1,2,4-Triazol als Ausgangsstoffe, so kann der Verlauf des erfindungsgemäßen Verfahrens durch das folgende Formelschema veranschaulicht werden:

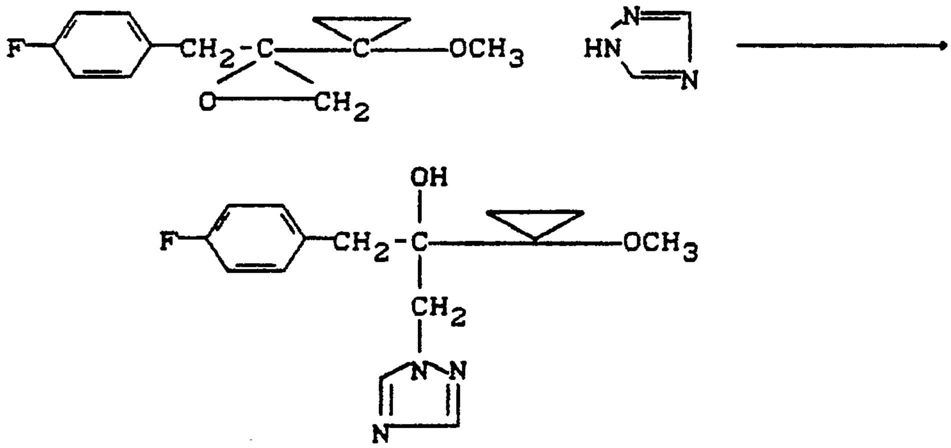

Die bei dem erfindungsgemäßen Verfahren als Ausgangsstoffe benötigten Oxirane sind durch die Formel (II) allgemein definiert. In dieser Formel haben R und X vorzugsweise diejenigen Bedeutungen, die bereits im Zusammenhang mit der Beschreibung der erfindungsgemäßen Stoffe der Formel (I) vorzugsweise für diese Reste genannt wurden.

Die Oxirane der Formel (II) sind bisher noch nicht bekannt. Sie lassen sich herstellen, indem man Cyclopropylketone der Formel

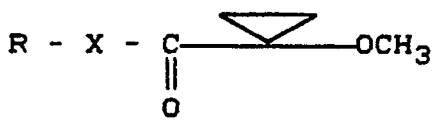 (IV),

in welcher
R und X die oben angegebenen Bedeutungen haben,
entweder

α) mit Dimethyloxosulfonium-methylid der Formel

$$\overset{\delta\oplus\,\delta\ominus}{(CH_3)_2SOCH_2} \qquad (V)$$

oder
β) mit Dimethylsulfonium-methylid der Formel

$$\overset{\delta\oplus\,\delta\ominus}{(CH_3)_2S\ CH_2} \qquad (VI)$$

in Gegenwart eines Verdünnungsmittels umsetzt.

Die Cyclopropylketone der Formel (IV) sind bisher ebenfalls noch nicht bekannt. Sie lassen sich herstellen, indem man N-Methoxy-N-methyl-1-methoxy-cyclopropan-carbonsäureamid der Formel

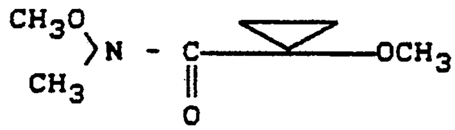 (VII)

entweder

γ) mit Grignard-Verbindungen der Formel

$$R-CH_2-MgX^1 \quad (VIII),$$

in welcher
R die oben angegebene Bedeutung hat und
$X^1$ für Chlor, Brom oder Iod steht,
in Gegenwart eines Verdünnungsmittels umsetzt,
oder

δ) mit Halogenverbindungen der Formel

$$R - Hal \quad (IX),$$

in welcher
R die oben angegebene Bedeutung hat und
Hal für Brom oder Iod steht,
in Gegenwart einer starken Base sowie in Gegenwart eines Verdünnungsmittels umsetzt.

Das N-Methoxy-N-methyl-1-methoxy-cyclopropan-carbonsäureamid der Formel (VII) ist bisher auch noch nicht bekannt. Es läßt sich herstellen, indem man 1-Methoxy-cyclopropan-carbonsäurechlorid der Formel

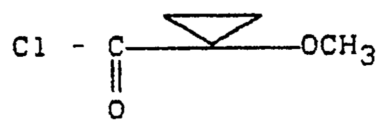 (X)

mit N,O-Dimethyl-hydroxylamin-Hydrochlorid der Formel

$$\begin{matrix}CH_3O\\CH_3\end{matrix}\rangle NH \times HCl \quad (XI)$$

in Gegenwart eines Säurebindemittels und in Gegenwart eines Verdünnungsmittels umsetzt.

Das 1-Methoxy-cyclopropan-carbonsäurechlorid der Formel (X) ist bisher ebenfalls noch nicht beschrieben worden. Es läßt sich herstellen, indem man 1-Methoxy-cyclopropan-carbonsäure der Formel

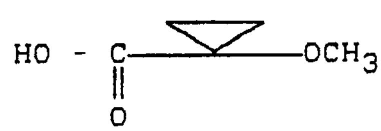 (XII)

mit Thionylchlorid gegebenenfalls in Gegenwart eines inerten Verdünnungsmittels bei Temperaturen zwischen 0°C und 100°C umsetzt. Die Aufarbeitung erfolgt nach üblichen Methoden.

Die bei den obigen Verfahren als Ausgangsstoffe bzw. Reaktionskomponenten benötigten Verbindungen der Formeln (VII), (IX), (XI) und (XII) sind bekannt (vgl. J. Amer. Chem Soc. 108, 2393 (1986)).

Bei dem obigen Verfahren zur Herstellung von N-Methoxy-N-methyl-1-methoxy-cyclopropan-carbonsäureamid kommen als Säurebindemittel vorzugsweise tertiäre aliphatische und aromatische Amine, wie Triethylamin, N,N-Dimethyl-cyclohexyl-amin, N,N-Dimethyl-benzylamin und Pyridin, und außerdem cyclische Amine, wie 1,5-Diaza-bicyclo-[4.3.0]-non-5-en (DBN), 1,8-Diaza-bicyclo[5.4.0]undec-7-en (DBU) und 1,4-Diaza-bicyclo[2.2.2]octan (DABCO) in Frage.

Als Verdünnungsmittel kommen bei dem obigen Verfahren zur Herstellung von N-Methoxy-N-methyl-1-methoxy-cyclopropan-carbonsäureamid der Formel (VII) alle für derartige Umsetzungen üblichen inerten organischen Solventien in Betracht. Vorzugsweise verwendbar sind halogenierte aliphatische Kohlenwasserstoffe, wie Dichlormethan, Chloroform und Tetrachlorkohlenstoff.

Die Reaktionstemperaturen können bei dem obigen Verfahren zur Herstellung von N-Methoxy-N-methyl-1-methcxy-cyclopropan-carbonsäureamid der Formel (VII) innerhalb eines bestimmten Bereiches variiert werden. Im allgemeinen arbeitet man bei Temperaturen zwischen -10 °C und +80 °C, vorzugsweise zwischen 0 °C und +60 °C.

Bei dem obigen Verfahren zur Herstellung von N-Methoxy-N-methyl-1-methoxy-cyclopropan-carbonsäureamid der Formel (VII) arbeitet man ebenso wie bei den anderen in dieser Anmeldung beschriebenen Verfahren im allgemeinen unter Normaldruck.

Bei dem obigen Verfahren zur Herstellung von N-Methoxy-N-methyl-1-methoxy-cyclopropan-carbonsäureamid der Formel (VII) setzt man auf 1 Mol an 1-Methoxy-cyclopropan-carbonsäurechlorid der Formel (X) eine äquivalente Menge oder auch einen Überschuß an Säurebindemittel ein. Die Aufarbeitung erfolgt nach üblichen Methoden. Im allgemeinen geht man so vor, daß man das Reaktionsgemisch filtriert und gegebenenfalls nach vorherigem Verdünnen mit einem in Wasser wenig löslichen organischen Solvens mit Wasser wäscht, dann trocknet, einengt und den verbleibenden Rückstand destilliert.

Bei der Variante (δ) des obigen Verfahrens zur Herstellung von Cyclopropylketonen der Formel (IV) kommt als starke Base vorzugsweise n-Butyllithium in Frage.

Als Verdünnungsmittel kommen bei der Durchführung der Variante (δ) des obigen Verfahrens zur Herstellung von Cyclopropylketonen der Formel (IV) alle für derartige Umsetzungen üblichen, inerten organischen Solventien in Betracht. Vorzugsweise verwendbar sind Ether, wie Diethylether.

Die Reaktionstemperaturen können bei der Durchführung der Variante (δ) des obigen Verfahrens zur Herstellung von Cyclopropylketonen innerhalb eines bestimmten Bereiches variiert werden. Im allgemeinen arbeitet man bei Temperaturen zwischen -80 °C und +40 °C.

Bei der Durchführung der Variante (δ) des obigen Verfahrens zur Herstellung von Cyclopropylketonen der Formel (IV) setzt man auf 1 Mol an N-Methoxy-N-methyl-1-meth-oxycyclopropan-carbonsäureamid eine äquivalente Menge oder auch einen Überschuß an Halogenverbindung der Formel (IX) sowie eine äquivalente Menge oder auch einen Überschuß an starker Base ein. Die Aufarbeitung erfolgt nach üblichen Methoden. Im allgemeinen geht man so vor, daß man das Reaktionsgemisch gegebenenfalls nach vorherigem Verdünnen mit einem organischen Solvens auf Eis-wasser gießt, dann mit einem in Wasser wenig löslichen organischen Lösungsmittel extrahiert, die vereinigten organischen Phasen trocknet und dann durch Abziehen des Verdünnungsmittels unter vermindertem Druck einengt.

Als Verdünnungsmittel kommen bei der Durchführung der Variante (γ) des obigen Verfahrens zur Herstellung von Cyclopropylketonen der Formel (IV) alle für derartige Umsetzungen üblichen inerten organischen Solventien in Betracht. Vorzugsweise verwendbar sind Ether, wie Diethylether, Tetrahydrofuran und Dioxan.

Die Reaktionstemperaturen können bei der Durchführung der Variante (γ) des obigen Verfahrens zur Herstellung von Cyclopropylketonen der Formel (IV) innerhalb eines bestimmten Bereiches variiert werden. Im allgemeinen arbeitet man bei Temperaturen zwischen -80 °C und +50 °C, vorzugsweise zwischen -80 °C und 40 °C.

Bei der Durchführung der Variante (γ) des obigen Verfahrens zur Herstellung von Cyclopropylketonen der Formel (IV) setzt man auf 1 Mol an N-Methoxy-N-methyl-1-methoxy-cyclopropan-carbonsäureamid der Formel (VII) im allgemeinen 1 bis 1,5 Mol an Grignard-Verbindung der Formel (VIII) ein, die zweckmäßigerweise unmittelbar zuvor hergestellt und in situ weiterverarbeitet wird. Die Aufarbeitung erfolgt nach üblichen Methoden. Im allgemeinen geht man so vor, daß man zunächst ansäuert und mit Wasser versetzt, dann die organische Phase abtrennt, wäscht und nach dem Trocknen einengt.

Das bei der Durchführung der Variante (α) des Verfahrens zur Herstellung von Oxiranen der Formel (II) als Reaktionskomponente benötigte Dimethyloxosulfonium-methylid der Formel (V) ist bekannt (vgl. J. Am. Chem. Soc. 87, 1363-1364 (1965)). Es wird bei der obigen Umsetzung in frisch zubereitetem Zustand verarbeitet, indem man es in situ durch Umsetzung von Trimethyloxosulfoniumiodid mit Natriumhydrid oder Natriumamid, insbesondere mit Kalium-tert.-butylat oder Natriummethylat, in Gegenwart eines Verdünnungsmittels erzeugt.

Das bei der Durchführung der Variante (β) des Verfahrens zur Herstellung von Oxiranen der Formel (II) außerdem als Reaktionskomponente in Betracht kommende Dimethylsulfonium-methylid der Formel (VI) ist ebenfalls bekannt (vgl. Heterocycles 8, 397 (1977)). Es wird bei der obigen Umsetzung ebenfalls in frisch hergestelltem Zustand eingesetzt, indem man es in situ z.B. aus Trimethylsulfonium-halogenid oder Trimethylsulfonium-methylsulfat, in Gegenwart einer starken Base, wie z.B. Natriumhydrid, Natriumamid, Natriummethylat, Kalium-tert.-butylat oder Kaliumhydroxid, in Gegenwart eines Verdünnungsmittels, wie

tert.-Butanol oder Dimethylsulfoxid erzeugt.

Als Verdünnungsmittel kommen bei der Durchführung des obigen Verfahrens zur Herstellung von Oxiranen der Formel (II) inerte organische Solventien in Frage. Vorzugsweise verwendbar sind Alkohole, wie tert.-Butanol, Ether, wie Tetrahydrofuran oder Dioxan, ferner aliphatische und aromatische Kohlenwasserstoffe, wie Benzol, Toluol oder Xylol, sowie stark polare Lösungsmittel, wie Dimethylsulfoxid.

Die Reaktionstemperaturen können bei der Durchführung des obigen Verfahrens zur Herstellung von Oxiranen der Formel (II )in einem größeren Bereich variiert werden. Im allgemeinen arbeitet man zwischen 0 °C und 100 °C, vorzugsweise zwischen 10 °C und 60 °C.

Bei der Durchführung des obigen Verfahrens zur Herstellung von Oxiranen der Formel (II) setzt man auf 1 Mol an Cyclopropyl-keton der Formel (IV) im allgemeinen 1 bis 3 Mol an Dimethyloxosulfonium-methylid der Formel (V) bzw. an Dimethylsulfonium-methylid der Formel (VI) ein. Die Isolierung der Oxirane der Formel (II) erfolgt nach üblichen Methoden.

Als Säurebindemittel kommen bei der Durchführung des erfindungsgemäßen Verfahrens alle üblichen anorganischen und organischen Basen in Frage. Vorzugsweise verwendbar sind Alkalicarbonate, wie Natrium- und Kaliumcarbonat, ferner Alkalimetallhydroxide, wie Natrium- und Kaliumhydroxid, außerdem Alkalimetallalkoholate, wie Natrium- und Kaliummethylat und -ethylat sowie Kalium-tert.-butylat, und weiterhin niedere tertiäre Alkylamine, Cycloalkylamine und Aralkylamine, wie insbesondere Triethylamin.

Als Verdünnungsmittel kommen bei der Durchführung des erfindungsgemäßen Verfahrens alle üblichen inerten organischen Solventien in Betracht. Vorzugsweise verwendbar sind Nitrile, wie Acetonitril, ferner aromatische Kohlenwasserstoffe, wie Benzol, Toluol und Dichlorbenzol, außerdem Formamide, wie Dimethylformamid, sowie stark polare Lösungsmittel, wie Dimethylsulfoxid und Hexamethylphosphorsäuretriamid.

Die Reaktionstemperaturen können bei der Durchführung des erfindungsgemäßen Verfahrens in einem größeren Bereich variiert werden. Im allgemeinen arbeitet man bei Temperaturen zwischen 0 °C und 200 °C, vorzugsweise zwischen 50 °C und 150 °C.

Bei der Durchführung des erfindungsgemäßen Verfahrens setzt man vorzugsweise auf 1 Mol an Oxiran der Formel (II) 1 bis 4 Mol an Azol der Formel (III) und 1 bis 2 Mol an Base ein. Die Isolierung der Endprodukte erfolgt in üblicher Weise.

Die erfindungsgemäßen Triazolylmethyl-cyclopropyl-Derivate der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie Xanthomonas oryzae;
Pseudomonas-Arten, wie Pseudomonas lachrymans;
Erwinia-Arten, wie Erwinia amylovora;

Pythium-Arten, wie Pythium ultimum;
Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;

Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform; Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Pyricularia oryzae und Pellicularia sasakii an Reis sowie zur Bekämpfung von Getreidekrankheiten, wie Leptosphaeria nodorum, Erysiphe und Pseudocercosporella. Außerdem zeigen die erfindungsgemäßen Stoffe eine sehr gute Wirkung gegen Venturia, Sphaerotheca und Botrytis. Ferner besitzen die erfindungsgemäßen Stoffe auch eine sehr gute in-vitro-Wirkung.

Schließlich üben die erfindungsgemäßen Stoffe auch eine herbizide Wirkung gegen Gräser aus.

Die erfindungsgemäßen Stoffe können in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktioniertenatürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie PolyoxyethylenFettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol,

Polyvinylacetat, sowie natürliche Phospholipide, wie Kephaline und Lecithine, und synthetische Phospholipide, Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die Herstellung und die Verwendung der erfindungsgemäßen Stoffe geht aus den folgenden Beispielen hervor.

Herstellungsbeispiele

Beispiel 1

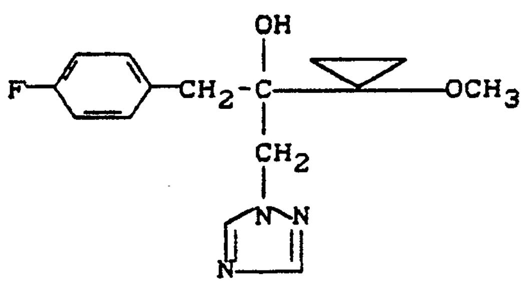

(I-1)

Ein Gemisch aus 1,5 g (6,8 mmol) 2-(4-Fluor-benzyl)-2-(1-methoxy-cyclopropyl)-oxiran, 1,5 g (21,7 mmol) 1,2,4-Triazol und 0,2 g (1,8 mmol) Kalium-tert.-butylat in 10 ml absolutem Dimethylformamid wird unter Stickstoffatmosphäre 8 Stunden auf 80 °C erhitzt. Danach wird das Lösungsmittel unter vermindertem Druck abgezogen und der verbleibende Rückstand in Essigsäureethylester gelöst. Die organische Lösung wird mit Wasser gewaschen, über Natriumsulfat getrocknet und unter vermindertem Druck eingeengt. Der Rückstand wird mit Dichlormethan als Laufmittel an Kieselgel chromatographiert. Nach dem Eindampfen des Eluates erhält man 0,45 g (23 % der Theorie) an 1-(4-Fluor-phenyl)-2-(1-methoxy-cyclopropyl)-3-(1,2,4-triazol-1-yl)-propan-2-ol in Form einer Festsubstanz mit dem Schmelzpunkt 72 °C.

Herstellung von Ausgangssubstanzen:

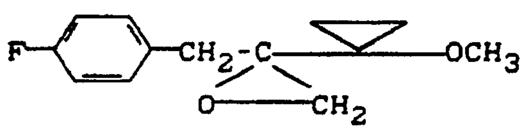

(II-1)

In ein Gemisch aus 2,2 g (10 mmol) Trimethylsulfoxoniumiodid und 0,4 g (13 mmol) Natriumhydrid (80 %ig) werden bei 10 °C unter Stickstoffatmosphäre 15 ml absolutes Dimethylsulfoxid eingetropft. Nach beendeter Zugabe läßt man innerhalb von 10 Minuten auf Raumtemperatur erwärmen und tropft dann unter Rühren eine Lösung von 2 g (9,6 mmol) 4-Fluorbenzyl-1-methoxycyclopropyl-keton in 10 ml absolutem Dimethylsulfoxid hinzu. Es wird zunächst 14 Stunden bei 20 °C und dann noch 1 Stunde bei 30 °C nachgerührt. Danach wird das Reaktionsgemisch auf Wasser gegossen. Das entstehende Gemisch wird mit Essigsäureethylester extrahiert. Die vereinigten organischen Phasen werden mit Wasser gewaschen, über Natriumsulfat getrocknet und durch Abziehen des Lösungsmittels unter vermindertem Druck eingeengt. Man erhält auf diese Weise 1,5 g (70 % der Theorie) 2-(4-Fluor-benzyl)-2-(1-methoxy-cyclopropyl)-oxiran in Form eines Öles.

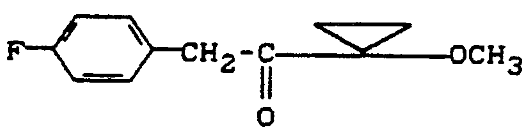

(IV-1)

In ein Gemisch aus 0,45 g (18,5 mmol) Magnesium-Spänen und 15 ml absolutem Ether werden 2,7 g (18,7 mmol) 4-Fluor-benzylchlorid so zugetropft, daß der Ether leicht siedet. Das Reaktionsgemisch wird 30 Minuten unter Rückfluß nachgerührt und anschließend auf -78 °C abgekühlt. Danach tropft man unter Rühren bei -78 °C 2 g (12,6 mmol) N-Methoxy-N-methyl-1-methoxycyclopropan-carbonsäureamid hinzu und läßt dann auf Raumtemperatur erwärmen. Das Reaktionsgemisch wird noch 2 Stunden bei Raumtemperatur gerührt und dann auf verdünnte, wäßrige Salzsäure gegossen. Man extrahiert das entstehende Gemisch mit Ether, wäscht die vereinigten organischen Phasen mit Wasser, trocknet über Natriumsulfat und engt durch Abziehen des Lösungsmittels unter vermindertem Druck ein. Es verbleiben 2,3 g (88 % der Theorie) an 4-Fluorbenzyl-1-methoxy-cyclopropyl-keton in Form eines Öles.

$^1$H-NMR (200 MHz, $CDCl_3$): $\delta$ = 1,1 - 1,33 (m, 4H); 3,38 (s, 3H); 3,97 (s, 2H),;6,92 - 7,15 (m, 2H); 7,15 - 7,25 (m, 2H).

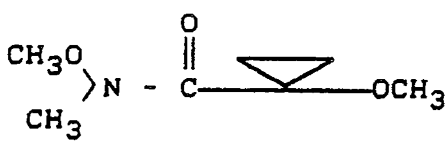

(VII)

In ein Gemisch aus 8 g (82 mmol) N,O-Dimethyl-hydroxylamin-Hydrochlorid und 100 ml absolutem Dichlormethan werden bei 20 °C unter Rühren 10 g (74 mmol) 1-Methoxy-cyclopropan-carbonsäurechlorid gegeben. Danach tropft man bei 0 °C 13 g (164 mmol) absolutes Pyridin hinzu. Man rührt zunächst eine Stunde bei 20 °C und dann eine Stunde bei 40 °C nach, filtriert dann den Niederschlag ab und versetzt mit 100 ml Dichlormethan. Das Reaktionsgemisch wird mit Wasser gewaschen, über Natriumsulfat getrocknet und durch Abziehen des Lösungsmittels unter vermindertem Druck eingeengt. Der verbleibende Rückstand wird einer Vakuumdestillation unterworfen. Man erhält auf diese Weise 10 g (85 % der Theorie) an N-Methoxy-N-methyl-1-methoxy-cyclopropan-carbonsäureamid in Form eines Öles mit einem Siedepunkt von Kp. = 125 °C bei 20 mbar.

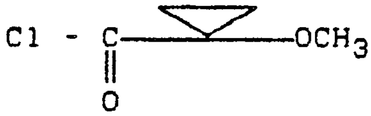

(X)

Zu einer Suspension von 61 g (0,52 mol) 1-Methoxy-cyclopropan-carbonsäure in 200 ml Dichlormethan werden bei Raumtemperatur 68 ml (0,78 mol) Thionylchlorid zugetropft. Das Reaktionsgemisch wird zwei Stunden unter Rückfluß gekocht, dann abgekühlt und durch Abziehen der flüchtigen Bestandteile unter vermindertem Druck eingeengt. Der verbleibende Rückstand wird im Wasserstrahlvakuum destilliert. Auf diese Weise erhält man 51,2 g (72 % der Theorie) an 1-Methoxy-cyclopropan-carbonsäure-chlorid in Form einer Flüssigkeit mit dem Siedepunkt 47 °C bei 18 mbar.

IR-Spektrum: Bande bei 1 780 $cm^{-1}$.

Beispiel 2

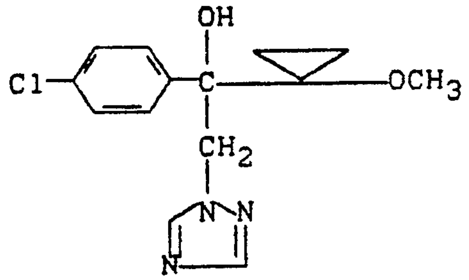

(I-2)

In eine auf 80° C erwärmte Lösung von 6,6 g (0,096 mol) 1,2,4-Triazol und 1,0 g (0,0096 mol) Kalium-tert.-butylat in 50 ml Dimethylformamid wird eine Lösung von 5,4 g (0,024 mol) 2-(1-Methoxy-cyclopropyl)-2-(4-chlorphenyl)-oxiran in 20 ml Dimethylformamid unter Rühren eingetropft. Das Reaktionsgemisch wird 12 Stunden bei 80° C gerührt, dann auf Raumtemperatur abgekühlt und unter vermindertem Druck eingeengt. Man löst den verbleibenden Rückstand in Ethylacetat, wäscht die entstehende organische Lösung dreimal mit Wasser, trocknet über Natriumsulfat und engt durch Abziehen des Lösungsmittels unter vermindertem Druck ein. Der Rückstand wird mit einem Gemisch aus Cyclohexan/Ethylacetat = 1:1 als Laufmittel an Kieselgel chromatographiert. Nach dem Eindampfen des Eluates erhält man 3,4 g (48 % der Theorie) an 2-(4-Chlor-phenyl)-2-(1-methoxycyclopropyl)-1-(1,2,4-triazol-1-yl)-ethan-2-ol in Form einer Festsubstanz mit dem Schmelzpunkt 106° C.

1H-NMR (250 MHz, $CDCl_3$): $\delta$ = 1,40-1,80 (m, 4H); 2,93 (s, 3H); 4,80 (2H, AB-System); 7,30 und 7,50 (2H, AB-System); 7,82 (s, 1H); 8,08 (s, 1H).

Herstellung von Ausgangssubstanzen:

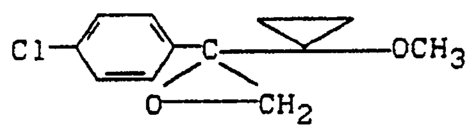

(II-1)

Eine Suspension aus 0,82 g (0,0342 mol) Natriumhydrid, 7,5 g (0,0342 mol) Trimethylsulfoxoniumiodid und 50 ml Dimethylsulfoxid wird zunächst 30 Minuten bei Raumtemperatur gerührt und dann bei 10° C mit einer Lösung von 6,0 g (0,0285 mol) 1-Methoxy-cyclopropyl-4-chlorphenyl-keton in 10 ml Dimethylsulfoxid versetzt. Nach beendeter Zugabe wird auf Raumtemperatur erwärmt und noch 3 Stunden gerührt. Man versetzt das Reaktionsgemisch dann mit 10 ml Ethylacetat und rührt weitere 5 Minuten. Man gießt das Reaktionsgemisch in Eiswasser, extrahiert das entstehende Gemisch dreimal mit Cyclohexan, trocknet die vereinigten organischen Phasen über Natriumsulfat und engt durch Abziehen des Lösungsmittels unter vermindertem Druck ein. Man erhält auf diese Weise 5,4 g (84 % der Theorie) an 2-(1-Methoxy-cyclopropyl)-2-(4-chlor-phenyl)-oxiran, das ohne zusätzliche Reinigung weiter umgesetzt wird.

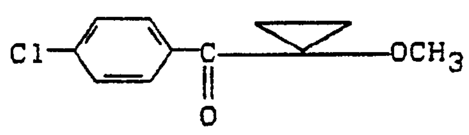

(IV-2)

Ein Gemisch aus 6,9 g (0,036 mol) 4-Bromchlorbenzol und 70 ml Diethylether wird unter Rühren bei -78° C mit 22 ml (0,036 mol) einer 1,6 molaren n-Butyl-lithium-Lösung versetzt. Man erwärmt das Reaktionsgemisch auf 0° C und rührt 15 Minuten bei dieser Temperatur. Nach erneutem Abkühlen auf -78° C wird eine Lösung von 4,8 g (0,03 mol) 1-Methoxycyclopropan-carbonsäure-N-methoxy-N-methylamid in 20 ml Diethylether schnell zugegeben. Danach wird jeweils eine Stunde bei -78° C, bei 0° C und bei Raumtemperatur nachgerührt. Anschließend versetzt man das Reaktionsgemisch mit 20 ml Ethylacetat, rührt 5 Minuten und gießt dann auf Eiswasser. Das entstehende Gemisch wird dreimal mit Ethylacetat

extrahiert, die vereinigten organischen Phasen werden über Natriumsulfat getrocknet und dann durch Abziehen des Lösungsmittels unter vermindertem Druck eingeengt. Man erhält auf diese Weise 6,1 g (97 % der Theorie) an 1-Methoxycyclopropyl-4-chlorphenyl-keton, das ohne vorherige Reinigung weiter umgesetzt wird.

$^1$H-NMR (250 MHz, $CDCl_3$): $\delta$ = 1,1-1,5 (m, 4H); 3,17 (s, 3H); 7,42 und 8,08 (2H, AB-System).

Nach den in den vorherigen Beispielen angegebenen Methoden werden auch die in den folgenden Beispielen aufgeführten Stoffe hergestellt.

Beispiel 3

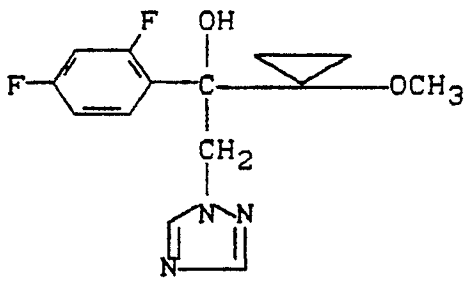

(I-3)

Schmelzpunkt: 107-108 °C

Beispiel 4

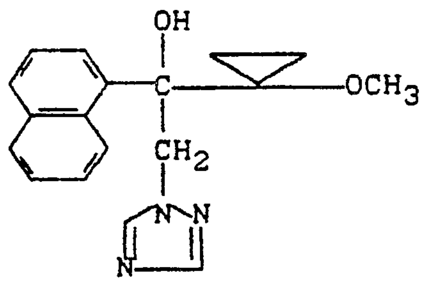

(I-4)

IR-Spektrum:
Banden bei 1595 $cm^{-1}$, 1700 $cm^{-1}$, 3100-3400 $cm^{-1}$.

In den folgenden Verwendungsbeispielen werden die nachstehend angegebenen Verbindungen als Vergleichssubstanzen eingesetzt:

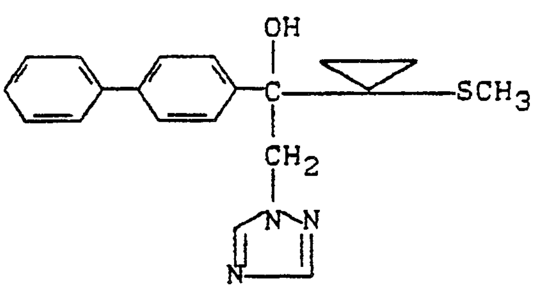

= (A)

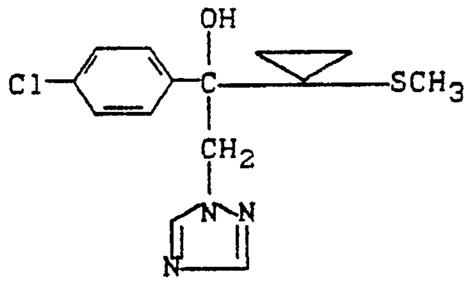

= (B)

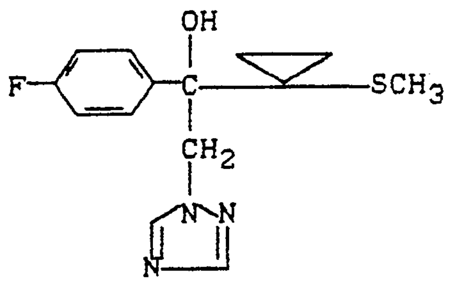

= (C)

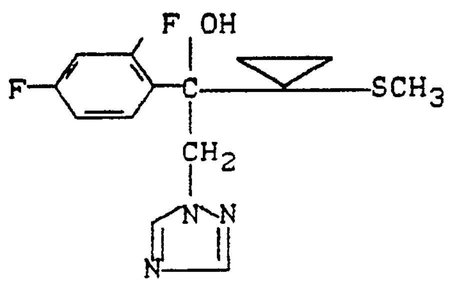

= (D)

Die Verbindungen sind aus der EP-OS 0 180 136 bekannt.

Beispiel A

Erysiphe-Test (Gerste)/protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigen die erfindungsgemäßen Verbindungen (1-2) und (1-4) eine wesentlich bessere Wirksamkeit als die Vergleichssubstanzen (A) und (B).

Beispiel B

Leptosphaeria nodorum-Test (Weizen) / protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit

den angegebenen Mengen Lösungsmittel und Emulgator und verdünnt das Konzentrat mit Wassr auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Sporensuspension von Leptosphaeria nodorum besprüht. Die Pflanzen verbleiben 48 Stunden bei 20° C und 100 % relativer Luftfeuchtigkeit in einer Inkubationskabine.

Die Pflanzen werden in einem Gewächshaus bei einer Temperatur von ca. 15° C und einer relativen Luftfeuchtigkeit von ca. 80 % aufgestellt.

10 Tage nach der Inokulation erfolgt die Auswertung.

In diesem Test zeigt die erfindungsgemäße Verbindung (1-1) eine wesentlich bessere Wirksamkeit als die Vergleichssubstanz (C).

Beispiel C

Pseudocercosporella herpotrichoides-Test (Weizen) / protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen an der Halmbasis mit Sporen von Pseudocercosporella herpotrichoides inokuliert.

Die Pflanzen werden in einem Gewächshaus bei einer Temperatur von ca. 10° C und einer relativen Luftfeuchtigkeit von ca. 80 % aufgestellt.

21 Tage nach der Inokulation erfolgt die Auswertung.

In diesem Test zeigt die erfindungsgemäße Verbindung (I-3) eine wesentlich besserte Wirksamkeit als die Vergleichssubstanz (D).

## Patentansprüche

1. Triazolylmethyl-cyclopropyl-Derivate der Formel

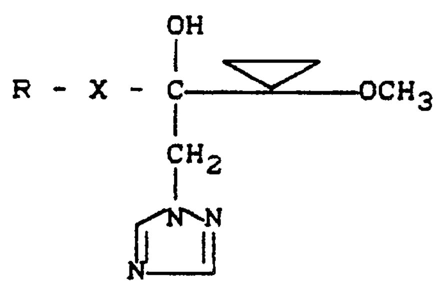

(I),

in welcher

R für die Reste der Formeln

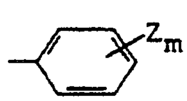

oder

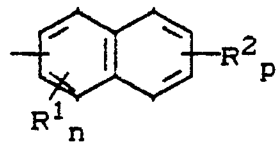

steht,

worin

Z für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenstomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht und

m für die Zahlen 0, 1, 2 oder 3 steht,

$R^1$ für Halogen, Alkyl mit 1 bis 4 Kohlenstoffatomen oder Alkoxy mit 1 bis 4 Kohlenstoffatomen steht,

n für die Zahlen 0, 1 oder 2 steht,

$R^2$ für Halogen, Alkyl mit 1 bis 4 Kohlenstoffatomen oder Alkoxy mit 1 bis 4 Kohlenstoffatomen steht und

p für die Zahlen 0, 1 oder 2 steht, und

X für $CH_2$ oder eine direkte Bindung steht, sowie deren Säureadditions-Salze und Metollsalz-Komplexe.

**2.** Verfahren zur Herstellung von Triazolylmethyl-cyclopropyl-Derivaten der Formel

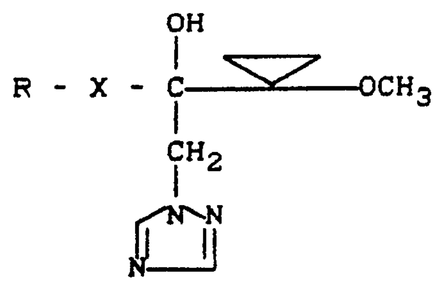

(I),

in welcher

R für die Reste der Formeln

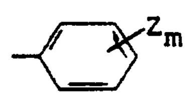

oder

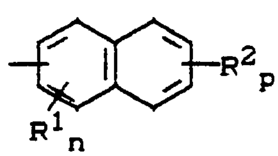

steht,

worin

Z für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht und

m für die Zahlen 0, 1, 2 oder 3 steht,

$R^1$ für Halogen, Alkyl mit 1 bis 4 Kohlenstoffatomen oder Alkoxy mit 1 bis 4 Kohlenstoffatomen steht,

n für die Zahlen 0, 1 oder 2 steht,

$R^2$ für Halogen, Alkyl mit 1 bis 4 Kohlenstoffatomen oder Alkoxy mit 1 bis 4 Kohlenstoffatomen steht und

p für die Zahlen 0, 1 oder 2 steht, und

X für $CH_2$ oder eine direkte Bindung steht,

sowie von deren Säureadditions-Salzen und Metallsalz-Komplexen, dadurch gekennzeichnet, daß man Oxirane der Formel

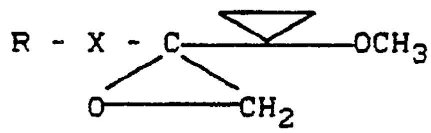

(II),

in welcher

R und X die oben angegebenen Bedeutungen haben,

mit 1,2,4-Triazol der Formel

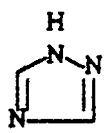

(III),

gegebenenfalls in Gegenwart eines Säurebindemittels und in Gegenwart eines Verdünnungsmittels umsetzt

und gegebenenfalls anschließend an die so erhaltenen Verbindungen der Formel (I) eine Säure oder ein Metallsalz addiert.

3. Fungizide Mittel, gekennzeichnet durch einen Gehalt an mindestens einem Triazolylmethyl-cyclopropyl-Derivat der Formel (I) gemäß Anspruch 1 bzw. an einem Säureadditions-Salz oder Metallsalz-Komplex eines Triazolylmethyl-cyclopropyl-Derivates der Formel (I).

4. Verwendung von Triazolylmethyl-cyclopropyl-Derivaten der Formel (I) gemäß Anspruch 1 bzw. von deren Säureadditions-Salzen und Metallsalz-Komplexen zur Bekämpfung von Pilzen.

5. Oxirane der Formel

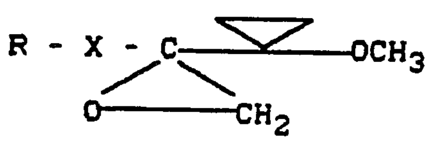

(II),

in welcher

R für die Reste der Formeln

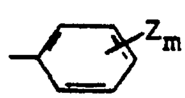

oder

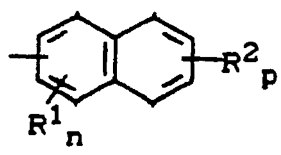

steht,
worin

Z für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht und

m für die Zahlen 0, 1, 2 oder 3 steht,

$R^1$ für Halogen, Alkyl mit 1 bis 4 Kohlenstoffatomen oder Alkoxy mit 1 bis 4 Kohlenstoffatomen steht,

n für die Zahlen 0, 1 oder 2 steht,

$R^2$ für Halogen, Alkyl mit 1 bis 4 Kohlenstoffatomen oder Alkoxy mit 1 bis 4 Kohlenstoffatomen steht und

p für die Zahlen 0, 1 oder 2 steht, und

X für $CH_2$ oder eine direkte Bindung steht.

**6.** Verfahren zur Herstellung von Oxiranen der Formel

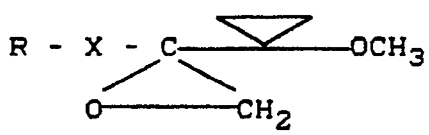

(II),

in welcher

R für die Reste der Formeln

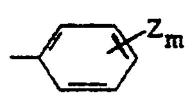

oder

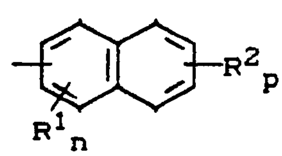

steht,
worin

Z für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Alkyl mit 1 oder 2 Kohlenstoffatomen und/oder Halogen substituiertes Phenyl

oder für gegebenenfalls durch Alkyl mit 1 oder 2 Kohlenstoffatomen und/oder Halogen substituiertes Phenoxy steht und

m für die Zahlen 0, 1, 2 oder 3 steht,

$R^1$ für Halogen, Alkyl mit 1 bis 4 Kohlenstoffatomen oder Alkoxy mit 1 bis 4 Kohlenstoffatomen steht,

n für die Zahlen 0, 1 oder 2 steht,

$R^2$ für Halogen, Alkyl mit 1 bis 4 Kohlenstoffatomen oder Alkoxy mit 1 bis 4 Kohlenstoffatomen steht und

p für die Zahlen 0, 1 oder 2 steht, und

X für $CH_2$ oder eine direkte Bindung steht,

dadurch gekennzeichnet, daß man Cyclopropyl-ketone der Formel

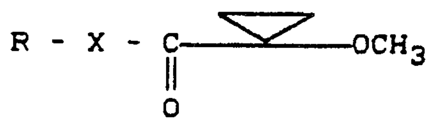

(IV),

in welcher

R und X die oben angegebenen Bedeutungen haben,

entweder

α) mit Dimethyloxosulfonium-methylid der Formel

$$\overset{\delta^{\oplus}\delta^{\ominus}}{(CH_3)_2SOCH_2} \qquad (V)$$

oder

β) mit Dimethylsulfonium-methylid der Formel

$$\overset{\delta^{\oplus}\delta^{\ominus}}{(CH_3)_2S\ CH_2} \qquad (VI)$$

in Gegenwart eines Verdünnungsmittels umsetzt.

7. Cyclopropyl-ketone der Formel

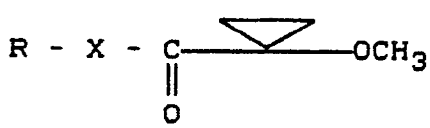

(IV),

in welcher

R für die Reste der Formeln

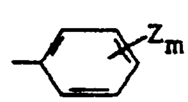

oder

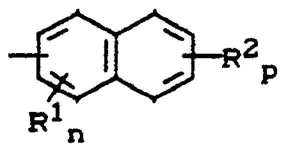

steht,
worin

Z für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht und

m für die Zahlen 0, 1, 2 oder 3 steht,

$R^1$ für Halogen, Alkyl mit 1 bis 4 Kohlenstoffatomen oder Alkoxy mit 1 bis 4 Kohlenstoffatomen steht,

n für die Zahlen 0, 1 oder 2 steht,

$R^2$ für Halogen, Alkyl mit 1 bis 4 Kohlenstoffatomen oder Alkoxy mit 1 bis 4 Kohlenstoffatomen steht und

p für die Zahlen 0, 1 oder 2 steht, und

X für $CH_2$ oder eine direkte Bindung steht.

**8.** Verfahren zur Herstellung von Cyclopropyl-ketonen der Formel

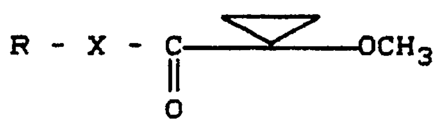

(IV),

in welcher

R für die Reste der Formeln

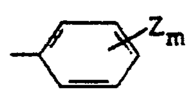

oder

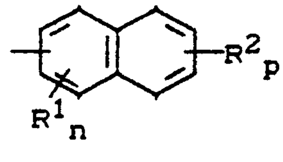

steht,
worin

Z für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht und

m für die Zahlen 0, 1, 2 oder 3 steht,

$R^1$ für Halogen, Alkyl mit 1 bis 4 Kohlenstoffatomen oder Alkoxy mit 1 bis 4 Kohlenstoffatomen steht,

n für die Zahlen 0, 1 oder 2 steht,

$R^2$ für Halogen, Alkyl mit 1 bis 4 Kohlenstoffatomen oder Alkoxy mit 1 bis 4 Kohlenstoffatomen steht und

p für die Zahlen 0, 1 oder 2 steht, und

X für $CH_2$ oder eine direkte Bindung steht,

dadurch gekennzeichnet, daß man N-Methoxy-N-methyl-1-methoxy-cyclopropan-carbonsäureamid der Formel

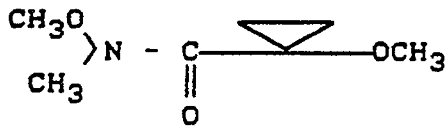

(VII)

entweder

γ) mit Grignard-Verbindungen der Formel

$$R-CH_2-MgX^1 \qquad (VIII),$$

in welcher

R die oben angegebene Bedeutung hat und

$X^1$ für Chlor, Brom oder Iod steht,

in Gegenwart eines Verdünnungsmittels umsetzt,

oder

δ) mit Halogenverbindungen der Formel

$$R - Hal \qquad (IX),$$

in welcher

R die oben angegebene Bedeutung hat und

Hal für Brom oder Iod steht,

in Gegenwart einer starken Base sowie in Gegenwart eines Verdünnungsmittels umsetzt.

**9.** N-Methoxy-N-methyl-1-methoxy-cyclopropan-carbonsäureamid der Formel

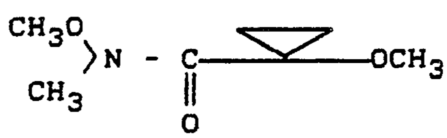

(VII) .

**10.** Verfahren zur Herstellung von N-Methoxy-N-methyl-1-methoxy-cyclopropan-carbonsäureamid der Formel

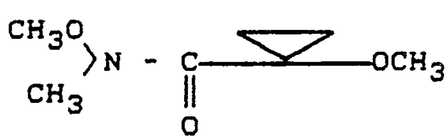

(VII) ,

dadurch gekennzeichnet, daß man 1-Methoxy-cyclopropan-carbonsäurechlorid der Formel

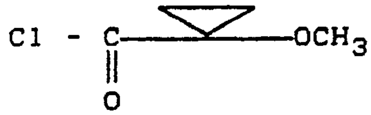 (X)

mit N,O-Dimethyl-hydroxylamin-Hydrochlorid der Formel

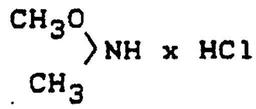 (XI)

in Gegenwart eines Säurebindemittels und in Gegenwart eines Verdünnungsmittels umsetzt.

**11.** 1-Methoxy-cyclopropan-carbonsäurechlorid der Formel

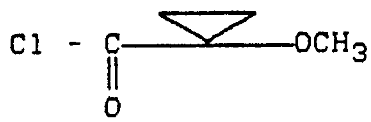 (X).

**12.** Verfahren zur Herstellung von 1-Methoxy-cyclopropan-carbonsäure-chlorid der Formel

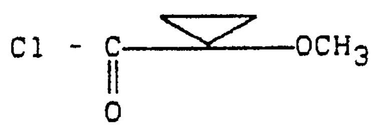 (X),

dadurch gekennzeichnet, daß man 1-Methoxy-cyclopropan-carbonsäure der Formel

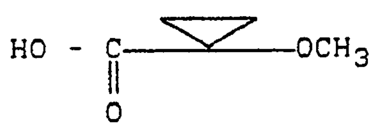 (XII)

mit Thionylchlorid gegebenenfalls in Gegenwart eines Verdünnungsmittels umsetzt.